# EUROPEAN PATENT APPLICATION

(11) **EP 4 656 134 A1**
(43) Date of publication of application: **03.12.2025**
(21) Application number: 23708522.0
(22) Date of filing: 24.01.2023
(51) Int. Cl.: A61B 5/15, A61M 5/32, A61B 17/34

(54) **MEDICAL DEVICE FOR GUIDING A NEEDLE TO A PUNCTURE POSITION**

(71) Applicant: ERRAZKIN TELLETXEA, Xabier, 20115 Astigarraga (ES)
(72) Inventor: ERRAZKIN TELLETXEA, Xabier, 20115 Astigarraga (ES)
(74) Representative: Galbaian S.Coop.
(86) International application number: PCT/ES2023/070037
(87) International publication number: WO 2024/156925

(57) **Abstract**

Medical device for guiding a needle to a puncture position, comprising a guiding platform (2) to allow a longitudinal movement of the needle on the guiding platform (2) to a puncture position (200), the guiding platform (2) being coupled to a base body (1) which is supported on a puncture area. The guiding platform (2) is pivotably attached to the base body (1), such that said guiding platform (2) can be fixed at different angles of inclination with respect to the base body (1).

## Description

### TECHNICAL FIELD

The present invention relates to puncture needle guiding devices for human or animal use.

### PRIOR ART

The use of sharp-pointed medical devices, such as puncture needles, scalpels, or a trocar, in humans or animals requires the practitioner to have the skill and dexterity to perform puncture manually. Depending on the type of needle, the angle of puncture is a determining factor in reaching the internal area of interest, avoiding possible undesired tears and injuries to the patient. Sometimes, guiding by means of visualizing the veins or areas where puncture is to be performed through ultrasound devices is also required, which further complicates the puncturing process.

US4403987A describes a device for supporting a hypodermic syringe and for facilitating injection of the needle of the hypodermic syringe into an arm or leg. The device includes a syringe supporting element and guiding means for supporting the movement of said element between a first position in which a needle supported on the syringe supporting member is retracted and a second injecting position. The guiding means include a pair of guide rods for supporting the syringe supporting element for slidable movement between the first position and the injecting position, and means for supporting the guiding means and for pinching flesh adjacent the syringe needle.

### DISCLOSURE OF THE INVENTION

The object of the invention is to provide a medical device for guiding a needle to a puncture position, as defined in the claims.

The medical device of the invention comprises a guiding platform to allow a longitudinal movement of the needle on the guiding platform to a puncture position, the guiding platform being coupled to a base body which is supported on a puncture area, wherein the guiding platform is pivotably attached to the base body, such that said guiding platform can be fixed at different angles of inclination with respect to the base body.

The device of the invention is configured to be used manually by the practitioner, allowing the practitioner to guide the needle over the puncture area of the body in a controlled manner, allowing the pointed device to be guided at a specific angle to avoid collateral damage due to undesired tearing of the vein.

In addition to being structurally simple and easy to manufacture, the device can be used together with visualization devices, such as an ultrasound device, which is extremely useful in ultrasound-directed vascular punctures.

These and other advantages and features of the invention will become apparent in view of figures and detailed description of the invention.

### DESCRIPTION OF THE DRAWINGS

Figure 1 shows a perspective view of the device according to an embodiment of the invention.
Figures 2 and 3 show a perspective view of the device of Figure 1 attached to an ultrasound device in two different usage positions of the device of the invention.
Figure 4 shows a view of an embodiment of the guiding platform of the device of the invention.
Figure 5 shows a section view of the base body together with a support and guiding means according to an embodiment of the invention.
Figure 6 shows a section view of the base body together with a support and guiding means according to another embodiment of the invention.
Figure 7 shows a schematic depiction of different angular positions of the support and the guiding means of the device with respect to the contact positions of the device according to an embodiment of the invention.
Figure 8 shows a schematic depiction of different angular positions of the support and the guiding means of the device with respect to the contact positions of the device according to another embodiment of the invention.

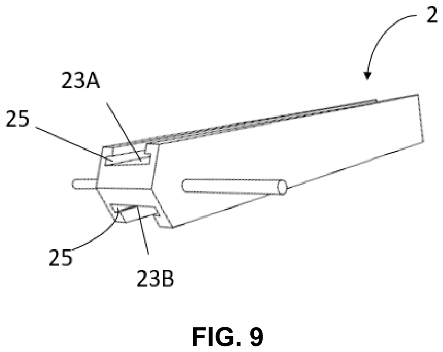

Figure 9 shows a perspective view of a guiding platform according to an embodiment of the invention.

### DETAILED DISCLOSURE OF THE INVENTION

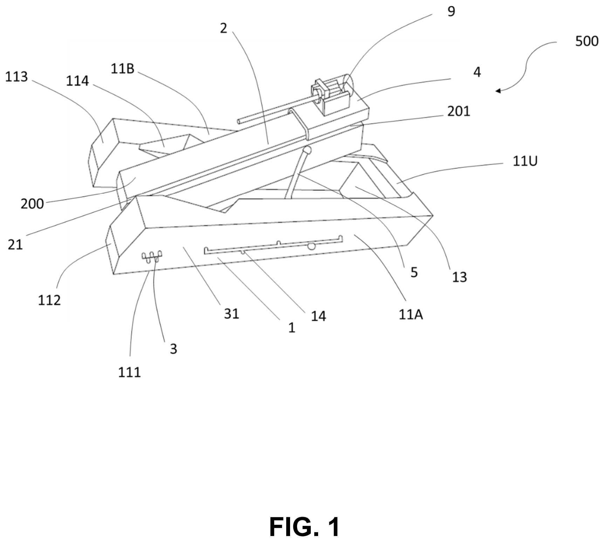

As shown in Figure 1, the device 500 for guiding a needle to a puncture position comprises a guiding platform 2 which allows a longitudinal movement of the needle on the guiding platform 2 from a retracted position 201 to a puncture position 200, the guiding platform 2 being coupled to a base body 1 configured for being supported on a puncture area, with the guiding platform 2 being pivotably attached to the base body 1, such that said guiding platform 2 can pivot and be fixed at different angles of inclination with respect to the base body 1. The user will be able to tilt or pivot the guiding platform at a specific angle with respect to the puncture area, depending on the angle of puncture required.

In the context of the invention, puncture area refers to the area of the animal or human body close to the area where the puncture should be made.

In the context of the invention, angle of puncture refers to the angle the pointed or puncture device, for example, the needle, must adopt depending on the puncture to be performed, i.e., intramuscular, subcutaneous, endovenous, or intradermal.

In the context of the invention, puncture position is understood as when the puncture device, more specifically the pointed element of the puncture device, for example, a needle tip, is in a contact position with the area where the puncture should be made.

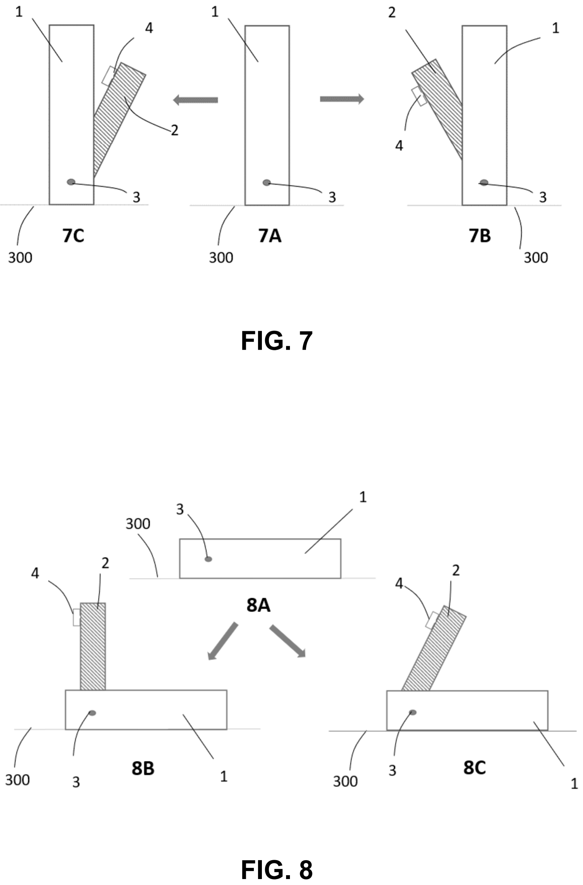

In one embodiment, the base body 1 can be arranged on the puncture area in a first position in which the guiding platform 2 is arranged parallel to the puncture area when it is arranged with an inclination of 0°, i.e., the base body 1 can be arranged on the puncture area in a first position in which the guiding platform 2 is arranged parallel to the puncture area 300 in the case where the guiding platform is arranged aligned with the base body 1, as shown in Figure 8A. In another embodiment, the base body is arranged on the puncture area in a second position in which the guiding platform 2 is arranged perpendicular to the puncture area when said guiding platform is arranged with an inclination of 0°, i.e., the guiding platform 2 is arranged perpendicular to the puncture area in the case where said guiding platform is arranged aligned with the base body 1, as shown in Figure 7 (see position 7A). In a preferred embodiment, the device can be arranged in a first position or a second position, according to practitioner's needs.

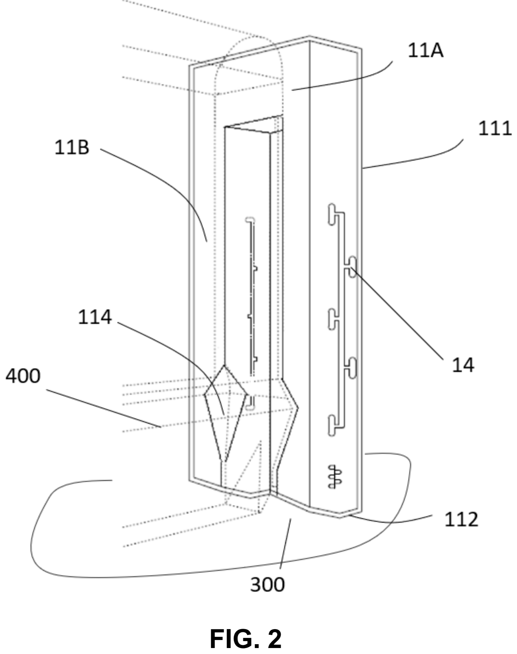

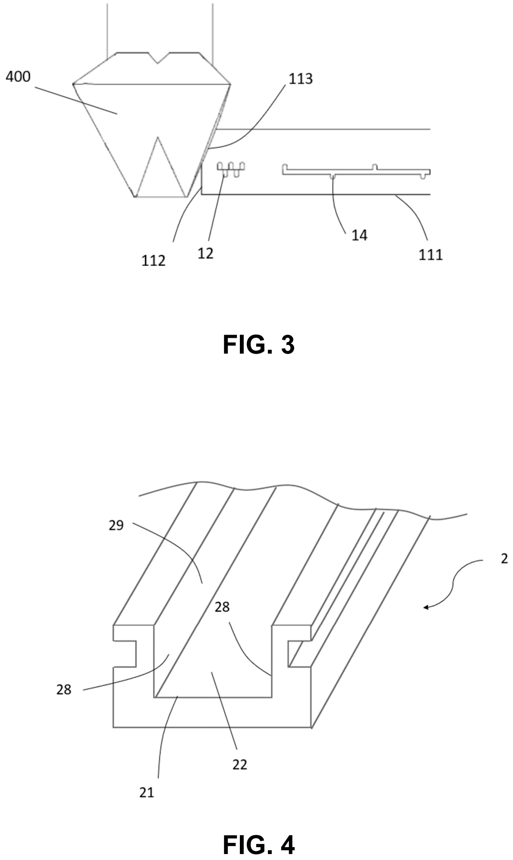

To better support the base body 1 on the puncture area 300, as shown in Figures 1 to 3, the base body 1 comprises in this embodiment at least one contact surface 111, 112 for being supported on a support surface of the puncture area 300. For example, contact surface 111 is used in the embodiment shown in Figure 3, whereas surface 112 is used in the embodiment shown in Figure 2. In another embodiment, the base body 1 comprises a second contact surface 113, 114 for being supported on a surface of an ultrasound imaging device, such as a probe of an ultrasound device 400 in Figure 2 (drawn with a dotted line) and Figure 3. This contact surface 113, 114 is useful when imaging by means of an ultrasound device is required to guide the puncture within the human body. This feature is useful in both the first position and the second position of the base body 1 described above, as shown in Figures 2 and 3. The guiding platform has been omitted in Figure 2 as it seeks to illustrate mainly the position of attachment with the ultrasound device.

In terms of the angles at which the guiding platform 2 pivots and is fixed, in one embodiment, the guiding platform 2 pivots and is fixed at an angle of at least between 0° and 180°, preferably between 0° and 90°, more preferably between 5° and 90°, with respect to the base body 1, when the base body 1 is arranged on the puncture area in the first position 8A. In another embodiment, the platform pivots and is fixed at an angle of at least between 0° and 90° or between 0° and -90°, more preferably at least between 5° and 90° or between -5° and -90°, with respect to the base body 1, when the base body 1 is arranged on the puncture area in the second position. In another embodiment, when it is not in any position, the platform pivots at an angle between 0° and 360°.

In one embodiment, the base body 1 comprises a rotating shaft 3, with one end 31 of the guiding platform 2 being coupled to said rotating shaft 3, the rotating shaft being configured to pivot the guiding platform 2 at a specific angle.

In one embodiment, the base body 1 comprises securing areas 12 for the rotating shaft 3 configured so that the guiding platform 2, in a specific angle of inclination, guides the needle to a specific area of the puncture area, this specific area being the area where the pointed element will perform puncture. Figure 3 illustrates an embodiment in which the base body 1 comprises notches 12 which secure the rotating shaft 3. Depending on the specific area to be punctured, the user will be able to fix the shaft in one notch or another.

In a preferred embodiment, as illustrated in Figures 1, 7, and 8, the guiding platform 2 pivots between a first position in which the guiding platform 2 is arranged in a housing 13 of the base body 1, thus being aligned with the base body 1, and a second position in which the guiding platform 2 is pivoted partially out of the base body 1. In the case of Figure 7, the base body 1 is supported on a contact surface 112, such that the housing 13 is perpendicular to the puncture area. In this embodiment, the guiding platform can adopt an angle of between 0 and 90° and between 0 and -90°, the last being useful for use thereof attached to probes of ultrasound devices. In the case of Figure 8, the base body 1 is supported on a contact surface 111, such that the housing 13 is horizontal to the puncture area. In this embodiment, the guiding platform can adopt an angle of between 0 and 180°. Figure 8 shows the transition to 90° with respect to the contact area.

In an embodiment of the invention as shown in Figures 1 and 2, the base body 1 comprises two parallel supports 11A, 11B comprising contact surfaces, with the rotating shaft 3 being arranged between both side supports 11A, 11B, preferably, between the ends of both side supports 11A, 11B. The housing 13 for the guiding platform 2 is arranged between both side supports. Additionally, the base body may have a third support 11U perpendicular to the side supports, the base body 1 adopting a U shape.

In another embodiment not shown in the figures, the base body 1 has two supports, said supports being attached in an L shape, with the rotating shaft 3 being arranged at a free end of one of the supports. The housing 13 is arranged between both supports.

In one embodiment, the guiding platform 2 is fixed by fixing means 5 which fix the guiding platform 2 with the base body 1 at a desired angle of inclination. Preferably, these fixing means are at least one rod which attaches the guiding platform 2 with the base body 1. The rod can be attached at different points 14 of the base body 1 such that the guiding platform 2 can be fixed in different angular positions. Figure 3 shows several anchoring notches or points 14 arranged in the base body 1, with the notches identifying the envisaged degree of inclination, and allowing the anchoring of one end of the rod to the notch, depending on the required angular position of the guiding platform 2, being preferably at 10°, 15°, 25°, 45°, and 90° with respect to the puncture area.

In another embodiment not shown in the figures, the fixing means consist of a gearwheel shaft, where this shaft may coincide with the rotating shaft 3.

With respect to the guiding platform 2, in a preferred embodiment, the platform comprises guiding means 29 and a surface 22 for sliding between a retracted position 201 and a puncture position 200 of the puncture needle. In one embodiment shown in Figure 4, the guiding platform 2 comprises a rectangular prism comprising at least one longitudinal channel 21 with a base 22 and walls 28, with the base 22 acting as a sliding surface and the walls 28 as guiding means 29.

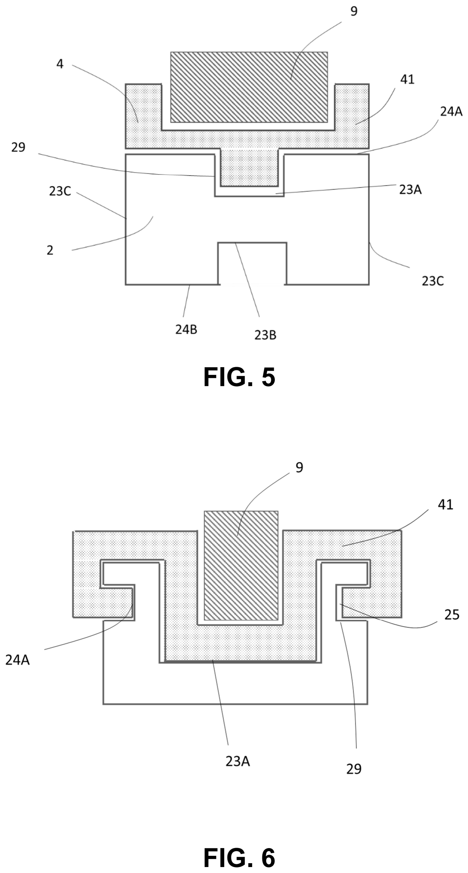

In another embodiment illustrated in Figure 5, the guiding platform 2 comprises a support 4, preferably a support plate 41, configured to retain at least one part 9 of the needle, for example, a rear chamber of a needle, the support 4 being arranged on or engaged in the guiding means 29, said guiding means 29 allowing the sliding of the support 4 in the longitudinal direction on said guiding means 29 between a retracted position 201 and a puncture position 200. In a preferred embodiment, the guiding means 29 comprise a longitudinal surface 24A, 24B and at least one longitudinal groove or recess 23A, 23B which is coupled to at least one portion of the support 4, slidingly retaining the support 4 between a retracted position 201 and a puncture position 200 of the support 4. Figures 5 and 6 show an example of two embodiments in which the guiding platform 2 comprises a rectangular prism comprising at least one longitudinal recess 23A on face 24A of the prism. Faces 23C adjacent to face 24A may comprise a longitudinal groove or recess 25, as shown in Figure 6. In another embodiment, the guiding means 29 comprise two longitudinal opposite surfaces 24A, 24B, with their corresponding recesses 23A, 23B, 25 or grooves. The user will therefore be able to choose to place the support 41 on face 24A or on face 24B, depending on whether they choose a positive angle (between 0° and 90°) or a negative angle (between 0° and -90°).

Preferably, the guiding platform 2, in the different embodiments thereof, allows movement of the needle from a retracted position 201 to a puncture position 200 and vice versa, being extremely useful in guiding catheter-type needles since, in these cases, once the catheter has been introduced with the needle, the needle must be removed carefully, avoiding the tearing of vein or tissue.

To better control the sliding of the needle or the support comprising the needle on the guiding platform 2, the guiding platform 2 may comprise a surface which generates a resistance to the free sliding of the needle or the support on the guiding platform, so it is necessary to exert a thrust force with the hand or finger on the needle. By way of example, this resistance can be achieved by means of a coarse surface or adapting the clearance of the recesses or grooves described above.

In one embodiment, the guiding platform 2 comprises an element for measuring the distance traveled by the needle during puncture. This feature, for example, is useful in ultrasound-directed punctures in which needle angulation and advancement control within the animal or human body is critical. The ultrasound device allows identifying the target structure to be punctured, as well as the depth of the target structure. The device of the invention allows fixing the angulation and knowing the distance traveled by the needle within the human body. In a preferred embodiment, the element for measuring is a meter. By way of example, the guiding means 29 and/or the surface 22 of the guiding platform may comprise distance marks such as the distance marks of a ruler. These marks allow the user to know the distance traveled by the puncture needle.

Although the device has been described for a needle, it can also work with any other sharp-pointed instrument, such as a scalpel or a trocar.

## Claims

1. Medical device for guiding a needle to a puncture position, comprising a guiding platform (2) to allow a longitudinal movement of the needle on the guiding platform (2) to a puncture position (200), the guiding platform (2) being coupled to a base body (1) which is supported on a puncture area (300), **characterized in that** the guiding platform (2) is pivotably attached to the base body (1), such that said guiding platform (2) can be fixed at different angles of inclination with respect to the base body (1).

2. Device according to claim 1, wherein the base body (1) can be arranged on the puncture area in a first position in which the guiding platform (2) is arranged parallel to the puncture area when it is arranged with an inclination of 0°, and in a second position in which the guiding platform (2) is arranged perpendicular to the puncture area when said guiding platform is arranged with an inclination of 0°.

3. Device according to claim 1 or 2, wherein the base body (1) comprises at least one contact surface (111, 112) for being supported on a support surface of the puncture area (300).

4. Device according to any of the preceding claims, wherein the base body (1) comprises a contact surface (113) for being supported on a surface of a probe of an ultrasound device.

5. Device according to any of claims 2 to 4, wherein the guiding platform (2) pivots and is fixed at an angle of between 0° and 180° with respect to the base body (1) when the base body (1) is arranged on the puncture area in the first position.

6. Device according to any of claims 2 to 5, wherein the guiding platform (2) pivots and is fixed at an angle of between 0° and 90° or 0° and -90° with respect to the base body (1) when the base body (1) is arranged on the puncture area in the second position.

7. Device according to any of the preceding claims, wherein the base body (1) comprises a rotating shaft (3), with one end (21) of the guiding platform (2) being coupled to said rotating shaft (3), the rotating shaft being configured to pivot the guiding platform (2) at a specific angle with respect to the base body (1).

8. Device according to claim 7, wherein the base body (1) comprises securing areas (12) for the rotating shaft (3) configured so that the guiding platform (2), at a specific angle of inclination, guides the needle to a specific area of the puncture area (300).

9. Device according to claim 7 or 8, wherein the base body (1) comprises two parallel supports (11A, 11B) comprising the contact surface, with the rotating shaft (3) being arranged between both side supports (11A, 11B).

10. Device according to claim 7 or 8, wherein the base body (1) has two supports comprising the contact surface, said supports being attached in an L shape, with the rotating shaft (3) being arranged on one of the supports.

11. Device according to any of the preceding claims, wherein the guiding platform (2) pivots between a first position in which the guiding platform (2) is arranged in a housing (13) of the base body (1), and a second position in which the guiding platform (2) is pivoted partially out of the base body (1).

12. Device according to any of the preceding claims, wherein the guiding platform (2) is fixed by fixing means (5), the fixing means (5) comprising at least one rod which attaches the guiding platform (2) with the base body (1).

13. Device according to claim 12, wherein the rod can be attached at different points (14) of the base body (1), such that the guiding platform (2) can be fixed in different angular positions.

14. Device according to claim 13, wherein the base body (1) comprises anchoring points (14) for the anchoring of the fixing means (5) for an angular position of the guiding platform (2), preferably at 10°, 15°, 25°, 45°, and 90°.

15. Device according to any of the preceding claims, wherein the guiding platform (2) comprises guiding means (29) and at least one surface (22) for sliding between a retracted position (201) and a puncture position (200) of the puncture needle.

16. Device according to any of the preceding claims, wherein the guiding platform (2) comprises a rectangular prism comprising at least one longitudinal channel (21) with a base (22) and walls (28), with the base (22) acting as a sliding surface and the walls (28) as guiding means (29).

17. Device according to any of claims 1 to 15, wherein the guiding platform (2) comprises a support (4), preferably a support plate (41), configured to retain at least one part of a needle (9), the support (4) being arranged on the guiding means (29), said guiding means (29) allowing the sliding of the support (4) in the longitudinal direction on said guiding means (29) between a retracted position (201) and a puncture position (200).

18. Device according to claim 17, wherein the guiding means (29) comprise a longitudinal surface (24A) and at least one longitudinal groove or recess (23A) which is coupled to at least one portion of the support (4), slidingly retaining the support (4), between a retracted position (201) and a puncture position (200) of the support plate (41).

19. Device according to claim 18, wherein the guiding means (29) comprise two longitudinal opposite surfaces (24A, 24B).

20. Device according to any of claims 17 to 19, wherein the support (4) is configured to retain a rear chamber of the needle.

21. Device according to any of the preceding claims, wherein the guiding platform (2) comprises an element for measuring the distance traveled by the needle during puncture.

22. Device according to claim 21, wherein the element for measuring is a meter.
